# EUROPEAN PATENT APPLICATION

(11) **EP 1 271 149 A2**
(43) Date of publication of application: **02.01.2003**
(21) Application number: 02013995.2
(22) Date of filing: 26.06.2002
(51) Int. Cl.: G01N 33/543, C12Q 1/68, G01N 33/52

(54) **Structure with immobilized biological material and method for manufacturing the same**

(30) Priority: 27.06.2001 JP 2001194786
(71) Applicant: FUJI PHOTO FILM CO., LTD., Kanagawa-ken, 250-0193 (JP)
(72) Inventor: Shinoki, Hiroshi, Asaka-shi, Saitama 351-8585 (JP); Seshimoto, Osamu, Asaka-shi, Saitama 351-8585 (JP)
(74) Representative: Albrecht, Thomas, Dr.

(57) **Abstract**

The present invention provides a structure comprising a vorticosely fixed string-like support, and plural regions on said string-like support to which a biological material is fixed. By using the structure according to the present invention, a target oligonucleotide having complementarity to an oligonucleotide can efficiently be detected.

## Description

### Technical Field

This invention relates to a structure in which a biological material such as DNA or protein (particularly, a specifically binding partner) is fixed, which is extremely useful for the analysis of expression, mutation, polymorphism and the like of the gene or the proteomics analysis, and to a method for manufacturing the same.

### Background Art

Recently, the technological development for efficiently analyzing gene functions of a variety of organisms has been rapidly progressed. For example, a detection tool called as a DNA chip where nucleotide derivatives such as a large number of DNA fragments or synthesized oligonucleotides are fixed on the surface of a solid phase substrate has been employed in order to analyze the nucleotide sequence of the DNAs or DNA fragments. A molecule for detection such as a DNA or its fragments or a synthesized oligonucleotide like a nucleotide derivative bound to the surface of such a solid phase substrate is also referred to as a probe molecule. A representative DNA chip is a microarray in which a large number of probe molecules are aligned and fixed to a solid support such as a slide glass or the like. DNA chip related technologies relating to manufacturing of a DNA chip and its use are considered to be capable of also utilizable for detecting a biomolecules other than DNA. Therefore, these are expected to provide a new means for aiming at drug discovery research, development of a method of diagnosis of diseases or preventing the disease, or the like.

The DNA chip related technologies has been materialized since the fact that the method for determining the nucleotide sequence of a DNA by hybridization with an oligonucleotide was developed. Although this method could overcome the limitation of the method for determining the nucleotide sequence using gel electrophoresis, initially it did not come into practice.

Subsequently, by developing the DNA chip configured as described above and its preparation technique, the expression, mutation, polymorphism or the like of a gene has been capable of efficiently being examined in a short period of time. Specifically, a DNA fragment sample showing the complementarity to a DNA fragment or an oligonucleotide on the DNA chip prepared (which is also referred to as a target DNA fragment) is, in general, detected by utilizing the hybridization of the DNA fragment or the oligonucleotide on the DNA chip with the labeled DNA fragment sample.

As for the method for preparing a DNA chip, two methods are known; that is to say, a method for directly synthesizing oligonucleotide on the surface of a solid support (referred to as "on-chip method") and a method in which DNA or oligonucleotide previously prepared is bound to the surface of the solid support [Science 270, 467-470 (1995)]. As for the former on-chip method, the representative method is comprised of selectively synthesizing oligonucleotide in a predetermined minute matrix region by combined use of a protective group which is selectively removed via photo irradiation, a photolithographic technology used for fabricating a semiconductor device and a technology for synthesizing a solid phase (referred to as "masking technology"). However, this method requires an expensive production devise and many steps of production process , and therefore great cost cutting per chip is difficult. Also, the latter method has many problems such as necessity for a special spotting device, impossibility of accurate spotting, difficulty in mass production, and the like.

As an example to solve the above-described problems, a method is described in Japanese Patent Laid-open Publication No. 2000-245460 such that DNA is fixed in a macaroni fiber, several macaroni fibers are bundled to form an accumulated body, and then the accumulated body is cut to prepare a thin chip body.

### Summary of the Invention

An object of the present invention is to provide a structure in which a biological material is fixed, which can be manufactured at a low cost and in a large scale. Another object of the present invention is to provide a method for manufacturing the above mentioned structure.

In order to achieve the above objects, the present inventors have studied earnestly, and have succeeded in obtaining a thin chip body to which oligonucleotide is fixed by the steps of dotting oligonucleotide onto a sheet material in a line, coating an adhesive material on the sheet, winding the sheet into the shape of a rod, and then cutting the rod-like substance into a thin chip body. Further, the present inventors have tried to detect oligonucleotide using this thin chip body and have demonstrated that DNA having a sequence complementary to the fixed oligonucleotide can be efficiently detected. The present invention has been accomplished based on these findings.

Thus, according to the present invention, there is provided a structure comprising a vorticosely fixed string-like support, and plural regions on said string-like support to which a biological material is fixed.

Preferably, the string-like support is obtained by cutting a sheet-like support. More preferably, the string-like support is obtained by cutting a rod-like substance formed by winding the sheet-like support in such a way that an end section of the wound sheet-like support appears vorticosely.

Particularly preferably, the structure of the present invention is obtained by the steps of; winding a sheet-like support to which plural lines of a biological material are fixed in such a way that the direction of said lines is the same as the direction of the centerline of the rod-like substance formed by the winding; and cutting the obtained rod-like substance in such a way that an end section of the wound sheet-like support appear vorticosely.

Preferably, the plural lines of a biological material are fixed to the sheet-like support at intervals of 10 µm to 1 cm.

Preferably, the thickness of the structure of the present invention ranges from 1 to 5000 µm.

Preferably, the biological material is a member of specific binding partners.

Preferably, the specific binding partners are antibody or antibody fragment/ligand, antibody or antibody fragment/antigen, antibody or antibody fragment/hapten, or receptor/ligand.

Preferably, the specific binding partners are avidins/biotins.

Preferably, the avidins are avidin, streptoavidin, or modified substance thereof that can form a stable complex with biotin.

Preferably, the biotins are biotin, biocytin, desthiobiotin, oxybiotin or derivatives thereof that can form a stable complex with avidin.

Preferably, the specific binding partners are nucleic acid/nucleic acid or nucleic acid/nucleic acid binding substance.

Preferably, the nucleic acid is oligonucleotide, polynucleotide or peptide nucleic acid.

Preferably, the nucleic acid binding substance is a double stranded DNA recognition substance.

Preferably, the double stranded DNA recognition substance is double stranded DNA recognition antibody, DNA transcription factor, protein having Zinc finger motif or Ring finger motif or peptide nucleic acid.

According to another aspect of the present invention, there is provided an analytical element and an analytical chip which comprise the above mentioned structure.

According to still another aspect of the present invention, there is provided a method for manufacturing a biological material-fixed structure comprising the steps of:
(1) winding a sheet-like support to which plural lines of a biological material are fixed in such a way that the direction of said lines is the same as the direction of the centerline of a rod-like substance formed by the winding; and
(2) cutting the rod-like substance obtained in the step (1) in such a way that an end section of the wound sheet-like support appear vorticosely.

### Brief Description of Drawing

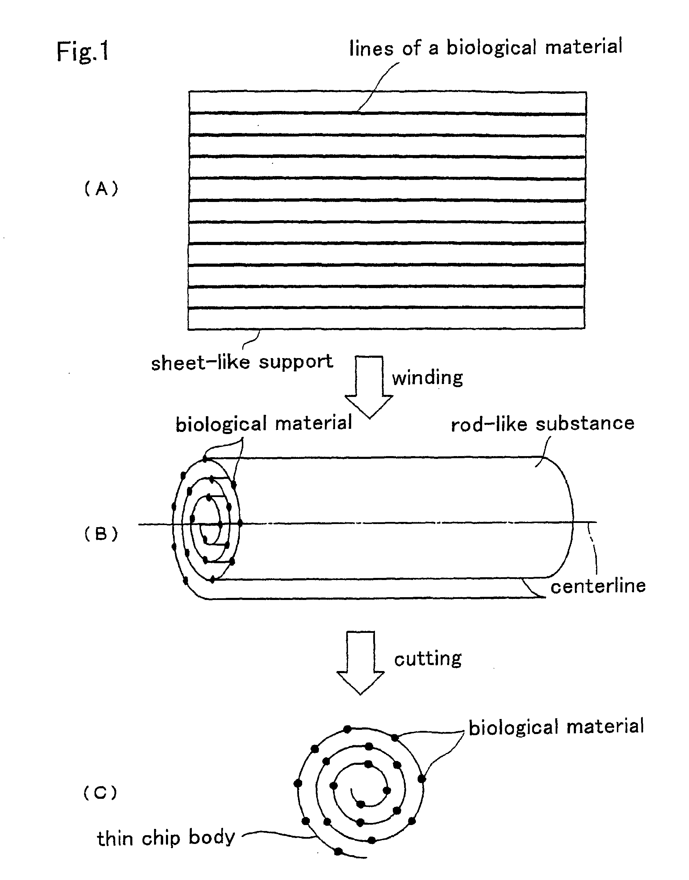

Fig. 1 is a schematic diagram which illustrates the process of the manufacturing method of the biological material-fixed structure according to the present invention.

### Detailed Description of the Invention

Hereinafter, embodiments of the present invention is described in detail.

The structure according to the present invention comprises a vorticosely fixed string-like support, and plural regions on said string-like support to which a biological material is fixed.

The vorticosely fixed string-like support is preferably obtained by cutting a sheet-like support, and more specifically it is obtained by cutting a rod-like substance formed by winding the sheet-like support in such a way that an end section of the wound sheet-like support appears vorticosely.

The structure according to the present invention can be formed, for example, by the steps of; winding a sheet-like support to which plural lines of a biological material are fixed in such a way that the direction of said lines is the same as the direction of the centerline of the rod-like substance formed by the winding; and cutting the obtained rod-like substance in such a way that an end section of the wound sheet-like support appear vorticosely. That is, the structure according to the present invention can be manufactured by a process of winding the sheet-like support and a process of cutting the thus formed rod-like substance . A schematic diagram of the process is shown in Fig. 1.

In Fig. 1, (A) in the upper side illustrates a sheet-like support to which plural lines of a biological material are fixed.

The sheet material which constitutes the sheet-like support is preferably a material which dose not exert a bad influence on the formation of binding between each member of specific binding partners, and has a flexible form capable of being wound. Further, a substrate which has a surface of low smoothness with a convex and a concave can be used. Specific examples of material for a sheet-like support include polyester series such as polyethylene terephthalate, polybutylene terephthalate, cellulose acetate, polycarbonate of bisphenol A, polystyrene, polymethylmethacrylate, nylon 6, nylon 66, polylactic acid, and polyglycolic acid; acrylic series such as polyacrylonitril; polyolefin series such as polyethylene and polypropylene; polyvinyl alcohol series; polyvinyliden chloride series; vinyl chloride series; polyurethane series; phenol series; fluorine-containing series including polyvinylidenfluoride and polytetrafluoroethylene; polyalkylene paraoxibenzoate series; derivative series of cellulose series made from diacetate, triacetate, chitin, chitosan or the like as material; protein series referred to as promix; cellulose series obtained by a viscose process, a cupper-ammonia process or an organic solvent process (rayon, cupra, polynosic or the like); flax, ramie, jute or the like.

Moreover, various types of substrates such as an electrode substrate used for an electrochemical analyzing method, a substrate for surface plasmon resonance (SPR) biosensor, and a charge coupled device, may also be utilized in a sheet-like shape. As a typical example, a sheet-like substance made of a thin membrane of gold or silver can be used. Further, these sheet-like materials can be used alone, and they can also be used with a coating of a reagent that helps a biological material to bind to the sheet-like material. In the case where the biological material has a minus charge (for example, DNA or its derivatives), an amine-based substance having a plus charge may be coated as the reagent. Furthermore, a functional group capable of forming covalent bond with the biological material may also be coated on the sheet-like material. Examples of such a functional group include succiimido, maleimido, acid anhydride, carbodiimido, mercapto, and vinylsulfone, but are not limited thereto. Any means can be utilized which can fix the biological material such as DNA, protein or hapten on the solid support.

The methods for forming lines of biological material on the sheet-like material include a slit coating method, a spotting method and an ink jet method.

In the case where the biological material is protein, a spotter device can be used. However, there is a possibility of lowering the activity of the protein depending on the property of a pinhead. In this case, use of an ink jet device or the like may be preferable. In order to prevent drying and denaturalization of the protein after formation of the line, a material having a high boiling point may be added, as is the case with nucleotide derivatives or their analogs.

The width of the line can be determined in accordance with the resolving power of a detecting device and is not particularly limited. In the case of a micro array, 100 µm will be sufficient.

As to the distance between the lines, in the case where many types of the biological material are used, a smaller thin body can be formed by making distance between the lines closer. Though the distance between the lines is not particularly limited, it ranges from 10 µm to 1 cm in general.

In the present invention, the rod-like substance is formed by winding the sheet-like support to which several lines of the aforementioned biological material are fixed. In Fig. 1, (B) at the mid side illustrates the thus formed rod-like substance. The winding of the support for forming the rod-like substance is carried out in such a way that the direction of the lines fixed on the sheet-like support is the same as the direction of the centerline of the rod-like substance. The centerline referred herein means the line which runs through the center of the convolution of the rod-like substance (pipe-like substance), which is the line referred to as the centerline in Fig. 1.

By providing an adhesive agent to the surface of the support as pretreatment before winding, good reproducibility can be realized in a succeeding process to form a thin chip.

According to the present invention, the vorticosely fixed string-like support (herein also referred to as thin chip body) having plural regions to which a biological material is fixed can be prepared by cutting a rod-like substance formed by winding the sheet-like support as mentioned above, in such a way that an end section of the wound sheet-like support appears vorticosely. In Fig. 1, it is shown that the thin chip body in (C) can be manufactured by cutting the rod-like substance in (B). The thin chip body thus manufactured comprises the vorticosely fixed string-like support, and plural regions on said string-like support to which the biological material is fixed.

The methods for preparing the thin chip body include a method wherein a thin chip body is cut out by using a diamond wire cutting machine (Musashino Electronic Co.) or a microtome. The thickness of the thin chip body can be arbitrarily adjusted, and usually it ranges from 1 to 5000 µm, and preferably from 10 to 2000 µm.

The types of the biological material used in the present invention are not particularly limited, and any material can be used. The material having affinity with other material is preferred, and a member of specific binding partners is particularly preferred.

The types of the specific binding partners which can form a biological specific binding are not particularly limited, and include combinations such as an antibody or an antibody fragment/a ligand, an antibody or an antibody fragment/an antigen, an antibody or an antibody fragment/substance having an antigenic determinant such as a hapten, a receptor/a ligand, avidins/biotins, a nucleic acid/a nucleic acid, and a nucleic acid/a nucleic acid binding protein.

When the structure having a biological material fixed thereon which is manufactured according to the present invention is used as DNA chip, the specific binding partners are those which have certain binding strength and can be used accurately and repeatedly in the subsequent hybridization process.

Examples of biotins include biotin, biocytin, desthiobiotin, oxybiotin, or their derivatives that can form a stable complex with avidin. The phrase "can form a stable complex" means that a complex having a dissociation constant approximate to the dissociation constant of biotin-avidin complex (10⁻¹⁵M) can be formed. Examples of avidins include avidin, streptavidin, or these altered bodies that can form a stable complex with biotin. The phrase "a stable complex" also means the same matter as defined above with respect to biotins. Further, the altered body means a modified body or its fragment of naturally occurring avidin or streptavidin, or recombinants thereof.

Examples of the nucleic acid include, but are not limited to, nucleotide derivatives or their analogs. Representative examples include an oligonucleotide, a polynucleotide, and a peptide nucleic acid. The nucleotide derivative or its analog may be naturally occurring one (DNA, DNA fragment, RNA or RNA fragment and the like), or may be a synthetic compound. Moreover, nucleotide derivatives or their analogs include a variety of analogous compounds such as what is called an LNA having a crosslinking group at its sugar unit portion (J. Am. Chem. Soc. 1998, 120:13252-13253).

The nucleic acid binding substances include a double stranded DNA recognition substance, but are not limited thereto. The double stranded DNA recognition substances include a substance which recognizes a double stranded DNA and specifically binds to it. Examples of the double stranded DNA recognition substance include a DNA transcription factor, a mismatch repairing protein, a double stranded DNA recognition antibody, or a peptide nucleic acid. Furthermore, the double stranded DNA recognition substances include substances having Zinc Finger motif or Ring finger motif.

The DNA transcription factor is a substance that binds to the promoter region on gene and controls the transcription from DNA to mRNA (Takaaki, Tamura: Transcription Factor, published by Yodosha, Co., Ltd., 1995). Accordingly, it is known that the transcription factor specifically binds to a double stranded DNA of the specific sequence.

Among a large number of transcription factors, Zinc Finger Protein, that is, a transcription factor group having Zinc finger and Ring Finger motifs, shows a very high occurrence rate in eucaryote, and 1% of the genome seems to code for them. Plabo et al. have analyzed the tertiary structure of Zinc Finger motif and elucidated the mechanism of its binding to DNA (Science 252:809 (1991)). Further, Choo et al. have succeeded in preparing a Zinc Finger Protein group which binds to the specific sequence but which does not exist in the nature, by a gene recombinant method (Nature 372: 642 (1994), PNAS 91: 11163 (1994)). Furthermore, the Scripps Research Institute group has succeeded in preparing a novel Zinc Finger Protein group by Phage Display (PNAS 95: 2812 (1998); 96: 2758 (1999)). As described above, the DNA transcription factor group represented by Zinc Finger Protein originally has the nature of binding to a double stranded DNA, and according to the researches in recent years, it has been made possible to prepare a recombinant which recognizes a given DNA sequence. It is possible to efficiently capture a double stranded DNA on a support by fixing such proteins.

Besides these, the nucleic acid binding substances include a helix-loop-helix protein and a substance having an Ets domain.

The structure having a biological material (for example, a nucleotide derivative or analogue thereof) fixed thereon according to the present invention can be used as an analytical element or an analytical chip in monitoring of gene expression, nucleotide sequencing, mutation analysis, polymorphism analysis and the like. In the case of fixing a nucleic acid, the principle of the detection is based on the hybridization with the labeled sample nucleic acid as mentioned above.

As labeling methods, although a RI method and a non-RI method (fluorescence method, biotin method, chemiluminescence method or the like) are known, it is preferred to use fluorescence method in the present invention. As a fluorescent substance utilized for a fluorescence labeling, any can be used if it can bind to the basic portion of nucleic acid. For example, cyanine dye (e.g., Cy3, Cy5 or the like of Cy Dye™ series, which is commercially available), rhodamine 6G reagent, N-acetoxy-N2-acetylaminofluorene (AAF) or AAIF (iodine derivative of AAF) can be used.

As a nucleic acid fragment sample, usually, a nucleic acid fragment sample such as a DNA fragment sample or a RNA fragment sample whose sequence and function is not known is used.

It is preferable that a nucleic acid fragment sample is isolated from the cell or tissue sample of eucaryote for the purpose of examining the gene expression. In the case where the sample is a genome, it is preferably isolated from any given tissue sample except for red blood cell. It is preferable that any given tissue except for red blood cell is peripheral blood lymphocyte, skin, hair, sperm or the like. In the case where the sample is an mRNA, it is preferable that it is extracted from the tissue sample in which the mRNA is expressed. It is preferable that a labeled cDNA is prepared from mRNA by incorporating a labeled dNTP ("dNTP" means a deoxyribonucleotide in which the base is adenine (A) , cytosine (C), guanine (G) or thymine (T)) using reverse transcription reaction. As a dNTP, use of dCTP is preferable because of chemical stability. Although the amount of the mRNA required for one hybridization is different depending on the amount of liquid to be spotted, and the type of labeling material, it is several µg or less. It is desired that the nucleic acid fragment sample has been previously depolymerized in the case where a DNA fragment on the nucleotide derivative or its analog fixed solid support is an oligoDNA. In the case of a prokaryotic cell, since the selective extraction of an mRNA is difficult, it is preferable that the total RNA is labeled.

As for a nucleic acid fragment sample, for the purpose of examining the mutation and polymorphism of a gene, it is preferable to obtain it by performing PCR of the target region in a reaction system containing the labeled primer or the labeled dNTP.

It is preferable that hybridization is carried out by dotting an aqueous solution, which is previously pipetted into a 96 wells or 384 wells plastic plate, in which labeled nucleic acid fragment samples are dissolved or dispersed, to the solid support of the present invention to which nucleotide derivatives or its analogs are fixed. The preferable amount of the solution for dotting is in the range from 1 to 100 nL. The hybridization is preferably carried out in the temperature range from room temperature to 70°C, and for the period from 6 to 20 hours. After the completion of hybridization, it is preferable that washing are performed using a mixed solution of a surfactant and a buffer solution to remove unreacted nucleic acid fragment samples. As a surfactant, it is preferable to use sodium dodecyl sulfate (SDS). As a buffer solution, citrate buffer solution, phosphate buffer solution, borate buffer solution, Tris buffer solution, Good' buffer solution or the like can be used. It is particularly preferable to use citrate buffer solution.

The hybridization using the solid support to which nucleotide derivatives or its analogs are fixed is characterized in that the amount of usage of the labeled nucleic acid fragment samples can be decreased to a very minute amount. Therefore, it is necessary to set the optimal conditions of the hybridization depending on the length of chain of the nucleotide derivatives or its analogs fixed to the solid support and the types of the labeled nucleic acid fragment samples. For the analysis of gene expression, it is preferable that a hybridization for a long time period is performed so as to be capable of sufficiently detecting even a lower expressing gene. For the detection of a single nucleotide polymorphism, it is preferable that a hybridization for a short period is performed. Moreover, it is also characterized in that comparison or quantitative determination of the expression amount are made possible using a single solid support to which DNA fragments are fixed by previously preparing two types of the nucleic acid fragment sample labeled by fluorescent substances different from each other and using them in a hybridization at the same time.

The present invention will be more concretely described below by the following examples. However, these examples are offered only to help an easy understanding of the present invention, and are not intended to limit the scope of the present invention.

### Example

### 1. Preparation of a thin chip film

To a nylon membrane (Hibond N Plus, manufactured by Amersham Pharmacia Co.) (15 cm X 15 cm), two types of oligonucleotide manufactured by BIO DOT Co. and having the following sequence respectively (each solution of 10⁻⁵ M) were dotted one after the other to form 40 lines at intervals of 1 mm.

(1): 3'-TCCTCCATGTCCGGGGAGGATCTGACACTTCAAGGTCTAG-5' (SEQ ID
NO.1)

(2): 3'-TCCTCCATGTCCGGGGAGGATCTGACACTTGAAGGTCTAG-5' (SEQ ID
NO.2)

Polyurethane resin adhesive agent was thinly coated, and then the nylon membrane to which two types of oligonucleotide were fixed was wound by using a rod of teflon (trade mark) having a diameter of 2 mm as a center core. The obtained rod-like substance was cut into 200 µm thickness by using a microtome to obtain thin chip bodies having a section of a film laminate containing a nucleic acid fixed gel.

### 2. Detection of a complementary target oligonucleotide sample

A solution prepared by dispersing an oligonucleotide sample of 22-mer (CTAGTCTGTGAAGTTCCAGATC)(SEQ ID NO.3) to 5' end of which Cy5 was bound in a hybridization solution (a mixed solution of 4 X SSC and 10 wt% SDS) (200 µL), and the thin chip film obtained in the above-described (1), were put in a test tube. The test tube was sealed, and incubated for 20 hours. Then, the thin chip film was taken out of the test tube, washed with a mixed solution of 0.1 wt% SDS and 2 X SSC, a mixed solution of 0.1 wt% SDS and 0.2 X SSC and 0.2 X SSC in this order, and dried at room temperature. Fluorescence intensity of the surface of the thin chip film (section of the film laminate above-described) was detected with a fluorescence-scanning device. The obtained values are 1078 for perfect matching and 195 for miss matching.

From the results described above, it is understood that by use of the oligonucleotide-fixed thin chip film support according to the present invention, a target oligonucleotide sample such as a target DNA fragment sample which has complementarity to the oligonucleotide can be efficiently detected.

### Effect of the Invention

The present invention makes it possible to provide the structure to which a biological material is fixed, which can be produced in a large scale at a low cost, and the method for manufacturing the structure. By using the structure according to the present invention, a target oligonucleotide having complementarity to an oligonucleotide can efficiently be detected.

## Claims

1. A structure comprising a vorticosely fixed string-like support, and plural regions on said string-like support to which a biological material is fixed.

2. The structure according to claim 1, wherein said string-like support is obtained by cutting a sheet-like support.

3. The structure according to claim 1, wherein said string-like support is obtained by cutting a rod-like substance formed by winding the sheet-like support in such a way that an end section of the wound sheet-like support appears vorticosely.

4. The structure according to claim 1 which is obtained by the steps of; winding a sheet-like support to which plural lines of a biological material are fixed in such a way that the direction of said lines is the same as the direction of the centerline of the rod-like substance formed by the winding; and cutting the obtained rod-like substance in such a way that an end section of the wound sheet-like support appear vorticosely.

5. The structure according to claim 4 wherein said plural lines of a biological material are fixed to the sheet-like support at intervals of 10 µm to 1 cm.

6. The structure according to any of claim 1 wherein the thickness ranges from 1 to 5000 µm.

7. The structure according to claim 1 wherein the biological material is a member of specific binding partners.

8. The structure according to claim 7 wherein said specific binding partners are antibody or antibody fragment/ligand, antibody or antibody fragment/antigen, antibody or antibody fragment/hapten, or receptor/ligand.

9. The structure according to claim 7 wherein said specific binding partners are avidins/biotins.

10. The structure according to claim 9 wherein said avidins are avidin, streptoavidin, or modified substance thereof that can form a stable complex with biotin.

11. The structure according to claim 9 wherein said biotins are biotin, biocytin, desthiobiotin, oxybiotin or derivatives thereof that can form a stable complex with avidin.

12. The structure according to claim 7 wherein said specific binding partners are nucleic acid/nucleic acid or nucleic acid/nucleic acid binding substance.

13. The structure according to claim 12 wherein nucleic acid is oligonucleotide, polynucleotide or peptide nucleic acid.

14. The structure according to claim 12 wherein said nucleic acid binding substance is a double stranded DNA recognition substance.

15. The structure according to claim 14 wherein said double stranded DNA recognition substance is double stranded DNA recognition antibody, DNA transcription factor, protein having Zinc finger motif or Ring finger motif or peptide nucleic acid.

16. An analytical element and an analytical chip which comprise the structure according to claim 1.

17. A method for manufacturing a biological material-fixed structure comprising the steps of:
(1) winding a sheet-like support to which plural lines of a biological material are fixed in such a way that the direction of said lines is the same as the direction of the centerline of a rod-like substance formed by the winding; and
(2) cutting the rod-like substance obtained in the step (1) in such a way that an end section of the wound sheet-like support appear vorticosely.


SEQUENCE LISTING

<110> FUJI PHOTO FILM CO., LTD.

<120> STRUCTURE IN WHICH BIOLOGICAL MATERIAL IS FIXED AND
      METHOD FOR MANUFACTURING THE SAME

<130> 12399EP

<140>
<141>

<150> 2001-194786
<151> 2001-06-27

<160> 3

<170> PatentIn Ver. 2.1

<210> 1
<211> 40
<212> DNA
<213> Artificial Sequence

<220>
<223> Description of Artificial Sequence:oligonucleotide
      to prepare a chip film

<400> 1
tcctccatgt ccggggagga tctgacactt caaggtctag                    40


<210> 2
<211> 40
<212> DNA
<213> Artificial Sequence

<220>
<223> Description of Artificial Sequence:oligonucleotide
      to prepare a chip film

<400> 2
tcctccatgt ccggggagga tctgacactt gaaggtctag                    40


<210> 3
<211> 22
<212> DNA

<213> Artificial Sequence

<220>
<223> Description of Artificial Sequence:oligonucleotide
      sample of a 22-mer to 5' end of which Cy5 is bound

<400> 3
ctagtctgtg aagttccaga tc                                                22